(19) Europäisches Patentamt
European Patent Office
Office européen des brevets

(11) **EP 2 279 775 B1**

(12) **EUROPÄISCHE PATENTSCHRIFT**

(45) Veröffentlichungstag und Bekanntmachung des Hinweises auf die Patenterteilung:
**14.03.2012 Patentblatt 2012/11**

(51) Int Cl.:
***A61N 1/362*** *(2006.01)*

(21) Anmeldenummer: **10168723.4**

(22) Anmeldetag: **07.07.2010**

(54) **Herzstimulator zur Stimulation eines Ventrikels in Reaktion auf ein in dem kontralateralen Ventrikel erfasstes Ereignis**

Cardiac stimulator for stimulating one ventricle in response to an event sensed in the contralateral ventricle

Stimulateur cardiaque pour stimuler un ventricule en réponse à un événement ventriculaire controlatéral détécté

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **28.07.2009 US 228978 P**

(43) Veröffentlichungstag der Anmeldung:
**02.02.2011 Patentblatt 2011/05**

(73) Patentinhaber: **Biotronik CRM Patent AG**
**6341 Baar (CH)**

(72) Erfinder:
• **Vollkron, Michael**
**3021 Pressbaum (AT)**

• **Lippert, Michael**
**91522 Ansbach (DE)**

(74) Vertreter: **Lindner-Vogt, Karin L.**
**Biotronik SE & Co. KG**
**Corporate Intellectual Properties**
**Woermannkehre 1**
**12359 Berlin (DE)**

(56) Entgegenhaltungen:
DE-T2- 60 215 458    US-A1- 2004 215 257
US-A1- 2006 224 198    US-A1- 2008 009 909

**Beschreibung**

Die Erfindung betrifft einen implantierbaren Herzstimulator für die kardiale Resynchronisationstherapie (CRT) eines Herzens. Der Herzstimulator kann ein Herzschrittmacher oder ein implantierbarer Kardioverter/ Defibrillator (ICD) oder eine Kombination aus beidem sein, der in der Lage ist, beide Ventrikel eines Herzens zu stimulieren.

Typischerweise weist ein derartiger Herzstimulator wenigstens eine rechtsventrikuläre Sensing-Einheit und eine rechtsventrikuläre Stimulationseinheit sowie eine linksventrikuläre Sensing-Einheit und eine linksventrikuläre Stimulationseinheit auf. Diese Einheiten sind im Betrieb des Herzstimulators jeweils über Elektrodenleitungen mit an geeigneten Stellen im Herzen zu implantierenden Elektroden verbunden. Die Elektrodenleitung mit den Elektroden zum Erfassen elektrischer Potentiale im linken Ventrikel des Herzens sowie zur Abgabe linksventrikulärer Stimulationsimpulse sind typischerweise Bestandteil einer links-ventrikulären Elektrodenleitung, die durch den Koronarsinus eines Herzens verlegt wird und daher auch als Koronarsinuselektrodenleitung bezeichnet wird. Die linksventrikuläre Elektrodenleitung kann aber auch eine epikardiale oder endokardiale Elektrodenleitung zum linken Ventrikel sein. Die Elektroden zum Erfassen elektrischer Potenziale im rechten Ventrikel sowie zur Abgabe rechtsventrikulärer Stimulationsimpulse sind typischer Weise an einer rechtsventrikulären Elektrodenleitung befestigt, deren distales Ende bis in den Apex des rechten Ventrikels ragt. Die Elektrodenleitungen sind typischerweise an ihrem proximalen Ende über normierte Steckverbindungen mit einem entsprechenden Herzstimulator verbunden.

Die typischen Stimulationsmodi eines rechtsventrikulären Herzstimulators, wie beispielsweise VVI, VVD oder DDD, können als bekannt vorausgesetzt werden. Gleiches gilt für die Abgabe von Stimulationsimpulsen nur im Bedarfsfall (Demand-Schrittmacher), bei der die Abgabe eines Stimulationsimpulses an eine jeweilige Kammer eines Herzens dann unterbunden wird, wenn zuvor in einem entsprechenden Escape-Intervall eine jeweilige Eigenaktion (intrinsische Kontraktion) der jeweiligen Herzkammer über eine dieser Herzkammer zugeordnete Sensing-Einheit des Herzstimulators erfasst wurde. Diese an sich bekannten Konzepte können auch bei dem hier beschriebenen Herzstimulator verwirklicht sein.

Selbstständige Kontraktionen einer jeweiligen Herzkammer werden auch als intrinsische Ereignisse, Sense-Ereignisse oder Eigenaktionen bezeichnet. Sie können in an sich bekannter Weise durch entsprechende Sensing-Einheiten, die im Betrieb mit einer jeweiligen Elektrode zum Erfassen von Myokard-Potenzialen verbunden sind, erfasst werden. Damit eine jeweilige Sensing-Einheit nicht durch ein Potenzial infolge der Abgabe eines Stimulationsimpulses beeinträchtigt wird, sind Sensing-Einheiten typischerweise so ausgebildet, dass sie innerhalb einer kurzen Blanking-Zeit, die auf einen Stimulus folgt, keine Ereignisse erfassen. Die Abgabe eines Stimulationsimpulses wird auch als Pace-Ereignis bezeichnet.

Um eine vorhandene Dys-Synchronizität von rechtem und linkem Ventrikel zu korrigieren, werden bei heutigen Herzstimulatoren für die kardiale Resynchronisationstherapie immer beide Ventrikel elektrisch stimuliert, um eine definierte interventrikuläre Verzögerungszeit (VV delay, VVD) zu gewährleisten. Dies trifft selbst bei intakter AV-Überleitung zu, also bei natürlicher Überleitung eines eine Kontraktion auslösenden Reizes vom Atrium zum Ventrikel über den AV-Knoten. Dadurch verzichtet man auf eine effizientere Kontraktion des intrinsisch erregbaren Ventrikels sowie auf die damit verbundene natürliche Regulierung der AV-Verzögerung, die beispielsweise auch körperliche Anstrengung reflektiert.

In EP 2060299 ist ein Herzstimulator vorgeschlagen, der selbsttätig zwischen einem reinen rechtsventrikulären Stimulationsmodus und einem biventrikulären Stimulationsmodus der genannten Art umschalten kann.

Die Dokumente US 2008/009909 A1 und US 2004/215257 A1 offenbaren Herzstimulatoren zur Aufnahme von Signalen eines Ventrikels und zur Stimulation des kontralateralen Ventrikels.

Ziel der Erfindung ist es, einen Herzstimulator zu schaffen, der eine effiziente kardiale Resynchronisationstherapie (CRT) ermöglicht.

Erfindungsgemäß wird dieses Ziel durch einen implantierbaren Herzstimulator erreicht, der wenigstens eine erste Sensing-Einheit zum Erfassen von Eigenaktionen eines ersten Ventrikels aufweist, die einen Eingang aufweist, der mit einer ersten Elektrodenleitung verbunden oder zu verbinden ist und die ausgebildet ist, ein an ihrem Eingang anliegendes elektrisches Eingangssignal derart auszuwerten, dass die Sensing-Einheit wenigstens ein für eine Kontraktion des ersten Ventrikels typisches Signalmerkmal detektiert und ein entsprechendes Ausgangssignal erzeugt. Außerdem weist der Herzstimulator wenigstens eine Stimulationseinheit zum Stimulieren eines jeweils anderen, zweiten Ventrikels eines Herzens auf, die einen Ausgang aufweist, der mit einer zweiten Elektrodenleitung verbunden oder zu verbinden ist und die ausgebildet ist, auf ein Steuersignal hin einen ventrikulären Stimulationsimpuls zu erzeugen und über den Ausgang abzugeben. Eine Stimulationssteuereinheit ist mit der ersten Sensing-Einheit und der Stimulationseinheit verbunden und ausgebildet, Ausgangssignale der Sensing-Einheit zu verarbeiten und Steuersignale für die Stimulationseinheit zu erzeugen. Weiter ist die Stimulationssteuereinheit ausgebildet, aus erfassten ventrikulären Eigenaktionen R des ersten Ventrikels ein jeweils aktuelles intrinsisches RR-Intervall abzuleiten und aus dem so gebildeten RR-Intervall ein Verzögerungsintervall $\Delta$ zu bestimmen, welches mit einem ventrikulären Ereignis des ersten Ventrikels beginnt und zu dessen Ende die Stimulationssteuereinheit eine Stimulation des zweiten Ventrikels auslöst, sofern diese nicht inhibiert wird.

Der erste Ventrikel ist meist der rechte Ventrikel, kann aber (z.B. bei Rechtsschenkelblock) auch der linke Ventrikel sein.

Die Erfindung beruht auf der Erkenntnis, dass in dem Fall, in dem ein zur Resynchronisierung geeignetes Herz einen intrinsischen Rhythmus in einem Ventrikel besitzt, die Resynchronisation durch Stimulation im anderen Ventrikel allein zu erreichen ist. Solange die Steuereinheit des Herzstimulators einen intrinsischen Rhythmus in der ersten Herzkammer detektieren kann (d.h. kein AV-Block vorliegt), wird der Zeitpunkt der Stimulation in der zweiten Kammer nach der im Folgenden beschriebenen Methode bestimmt. Falls kein intrinsischer ventrikulärer Rhythmus mehr vorliegt (z.B. AV-Block, Sinus-Bradykardie), wird die Resynchronisation nach der herkömmlichen Methode durchgeführt (Stimulation in beiden Ventrikeln). Eine automatische Umschaltung ist für intermittierenden AV-Block vorzusehen.

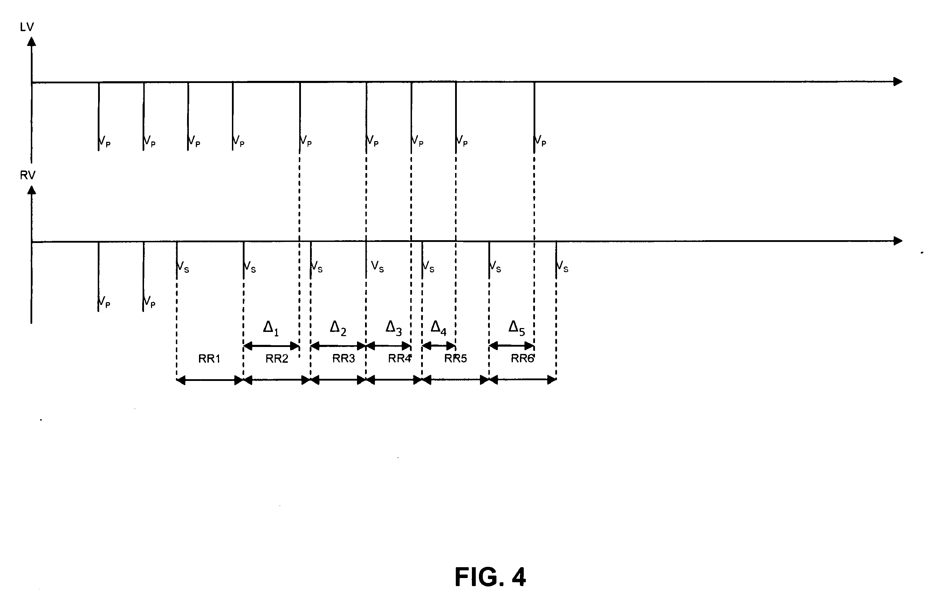

Die Resynchronisation wird in einem Herzstimulator gemäß der Erfindung durch ein Verzögerungsintervall Δ (siehe Figur 4), das Schlag für Schlag an RR, die Dauer des letzten intrinsischen RR-Intervalls, angepasst wird, erzielt. Wahlweise kann RR auch aus mehreren vorhergehenden RR-Intervallen berechnet werden. Der Stimulationszeitpunkt in einem der beiden Ventrikel folgt somit der jeweiligen Variabilität der Herzfrequenz und des intrinsischen AV-Delays.

Dazu ist durch eine geeignete Methode sicherzustellen, dass der zweite (asynchrone) Ventrikel derart stimuliert wird, dass er zugleich mit dem ersten (intrinsisch erregten) Ventrikel kontrahiert. In der Regel ist es dafür notwendig, den zweiten Ventrikel ein bestimmtes Zeitintervall VVD *vor* der Detektion im ersten Ventrikel (im selben Herzzyklus) zu stimulieren. Um diesen Zeitpunkt möglichst gut zu bestimmen, wird von der intrinsichen Erregung im *vorhergehenden* Herzzyklus ausgehend, nach Ablauf des Zeitintervalls Δ = RR - VVD im zweiten Ventrikel stimuliert.

Zu diesem Zweck ist es auch notwendig, eine Erregung des ersten Ventrikels durch atrioventrikuläre Überleitung (über den AV-Knoten) zu unterscheiden von einer interventrikulären Überleitung ausgehend vom (vorher stimulierten) zweiten Ventrikel.

Die Stimulationssteuereinheit ist dazu ausgebildet, das Verzögerungsintervall Δ unter Berücksichtigung einer vorbestimmen interventrikulären Verzögerungszeit VVD zu bestimmen. Eine Optimierung der Synchronisation kann dadurch mit Hilfe einer z.B. hämodynamischen Ersatzgröße durch die Anpassung des Parameters VVD [ms] durchgeführt werden. Dieser Parameter VVD gibt an, um wie viel der zweite (der zu resynchronisierende) Ventrikel vor der nächsten erwarteten intrinsischen Erregung im ersten Ventrikel stimuliert werden soll.

VVD kann hierzu einen vorbestimmten Defaultwert haben, der in einem Speicher des Herzstimulators gespeichert ist. Beispielsweise kann die interventrikuläre Verzögerung VVD während eines Follow-up bestimmt werden.

Die interventrikuläre Verzögerungszeit VVD kann manuell oder automatisch angepasst werden um ein hämodynamisches Optimum, abgeleitet aus z.B. intrakardialer Impedanz, Drucksensoren oder bildgebenden Verfahren, zu erreichen.

Falls ein negatives VVD (was einer Stimulation des zweiten Ventrikels erst nach der Detektion im ersten Ventrikel entspricht) notwendig ist, bzw. sich durch eine automatische Variation als Optimum ergibt, wird der zweite Ventrikel mit einer Verzögerung von - VVD nach der Detektion im ersten Ventrikel desselben Herzzyklus stimuliert. Ein RR-Wert wird in diesem Fall nicht benötigt.

Für den Fall, dass im ersten Ventrikel keine intrinsische Erregung detektiert wird, weil sie in die Blanking-Zeit nach der ventrikulären Stimulation fällt und dadurch eine direkte Bestimmung des Verzögerungsintervalls Δ nicht möglich ist, ist die Stimulationssteuereinheit ausgebildet,

- das Verzögerungsintervall Δ als Differenz zwischen der letzten intrinsischen Erregung und der Mitte des Blanking-Intervalls zu bestimmen;

- das Verzögerungsintervall Δ um einen Korrekturterm +delta oder -delta (delta so klein, dass kein wesentlicher Einfluss auf die Hämodynamik erwartet wird, z.B 20 ms) zu verändern, und zu prüfen, ob wieder eine intrinsische Erregung detektiert werden kann. Wenn das bei einem der Werte der Fall ist, wird dieser Korrekturterm beibehalten.

- Wenn das nicht zum Erfolg führt, wird eine Stimulation des zweiten Ventrikels für einen oder mehrere Herzschläge unterdrückt. Falls dann intrinsische Ereignisse im ersten Ventrikel auftreten, wird das intrinsische RR-Intervall, und ein neues Verzögerungsintervall Δ bestimmt. Falls kein intrinsisches Ereignis im ersten Ventrikel auftritt (z.B. AV-Block oder Sinus-Bradykardie, beides führt zu Stimulation im ersten Ventrikel) wird wegen fehlender intrinsischer Überleitung automatisch auf die normale biventrikuläre CRT-Stimulation umgeschaltet.

Außerdem ist die Stimulationssteuereinheit dazu ausgebildet, eine direkte Überleitung vom zweiten in den ersten Ventrikel dadurch zu detektieren, dass sie in regelmässigen Abständen das Verzögerungsintervall Δ für einen oder mehrere Herzzyklen um eine Zeitdauer τ verringert und die resultierende Veränderung des RR-Intervalls bestimmt. Wenn sich RR ebenfalls um τ in die gleiche Richtung verändert, detektiert die Stimulationssteuereinheit eine direkte Überleitung und reduziert das Verzögerungsintervall VVD.

Gemäß einer bevorzugten Ausführungsvariante erlaubt der implantierbare Herzstimulator ein Sensing in beiden Ventrikeln und bietet die Möglichkeit zur Stimulation in mindestens einem der beiden Ventrikel.

Außerdem ist ein implantierbarer Herzstimulator bevorzugt, bei dem die Blanking-Zeit nach einem ventrikulären Stimulus möglichst kurz ist, um das Verzögerungsintervall Δ auch im Fall von fast gleichzeitigen Sense- und Pace-Ereignissen noch mit ausreichender Genauigkeit bestimmen zu können.

Mit der Erfindung lassen sich eine Reihe von Vorteilen erzielen, nämlich:

- Die Nutzung der natürlichen Reizleitung im rechten Ventrikel führt zu einer effizienteren Kontraktion.

- Es wird die natürliche Anpassung AV Verzögerungszeit (AV delay) an verschiedene Belastungszustände genutzt.

- Die Erfindung bietet ein einfaches Konzept zur kontinuierlichen Anpassung der VV-Verzögerung bei intakter AV-Überleitung

- Die Erfindung ermöglicht das Erkennen von Überleitungen vom linken in den rechten Ventrikel.

- Die Erfindung erlaubt einen geringeren Energieverbrauch als herkömmliche biventrikuläre Herzstimulatoren durch eine Reduktion der Häufigkeit von RV Stimulation.

- Das vorgeschlagene Konzept bietet einen Lösungsvorschlag für eine konkrete klinische Fragestellung: "Warum sollen wir bei intakter Überleitung den rechten Ventrikel künstlich stimulieren?"

- Das vorgestellte erfindungsgemäße Konzept ist sehr gut geeignet, um in eine automatische AV/VV-Optimierung eingebunden zu werden. Ist der Herzstimulator - wie andernorts beschrieben - dazu ausgebildet, selbsttätig ein optimales AV delay (eine optimale atrioventrikuläre Verzögerungszeit) zu ermitteln, kann das zu einer optimalen AVD-Einstellung führen, die eine intrinsische Erregung im ersten Ventrikel zur Folge hat. In diesem Fall kann nahtlos auf die vorgestellte Methode übergegangen werden.

Die Erfindung soll nun anhand eines Ausführungsbeispiels unter Bezug auf die Figuren näher erläutert werden.

Diese zeigen in:

Figur 1: eine schematische Darstellung eines Herztherapiesystems;

Figur 2: eine Darstellung eines Herzstimulators mit angeschlossenen, im Herzen angeordneten Elektroden;

Figur 3: ein schematisches Blockschaltbild eines Herzstimulators;

Figur 4: eine beispielhafte Darstellung der zeitlichen Abfolge einer Stimulation.

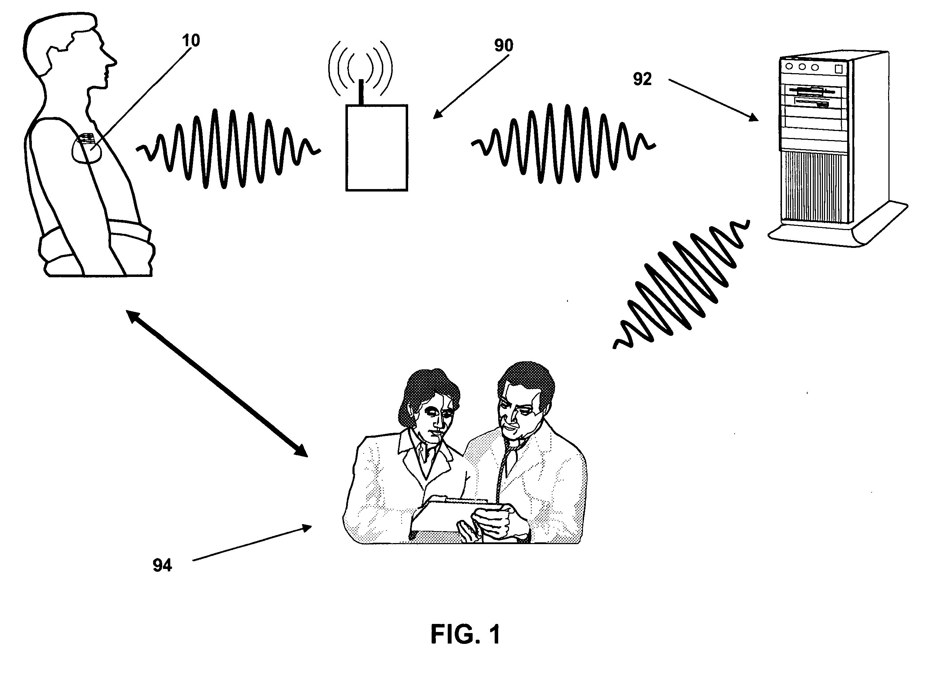

In Figur 1 ist zur Übersicht ein Herztherapiesystem dargestellt, das neben einem implantierten Herzschrittmacher 10 ein externes Gerät (Patientengerät) 90 sowie ein durch einen Server symbolisch dargestelltes Servicecenter 92 umfasst. Der implantierbare Herzstimulator 10 besitzt eine Telemetrie-Einheit, für die er drahtlos Daten mit dem externen Gerät 90 austauschen kann. Das externe Gerät 90 ist beispielsweise drahtgebunden mit dem Servicecenter 92 verbunden, so dass insgesamt Daten zwischen dem Servicecenter 92 und dem implantierbaren Herzstimulator 10 über das externe Gerät 90 als Relaisstation ausgetauscht werden können. Ein Ärzteteam 94 kann über einen datentechnischen Zugriff auf das Servicecenter 92 Einsicht in Daten nehmen, die das Servicecenter 92 von dem implantierbaren Herzstimulator 10 erhalten hat.

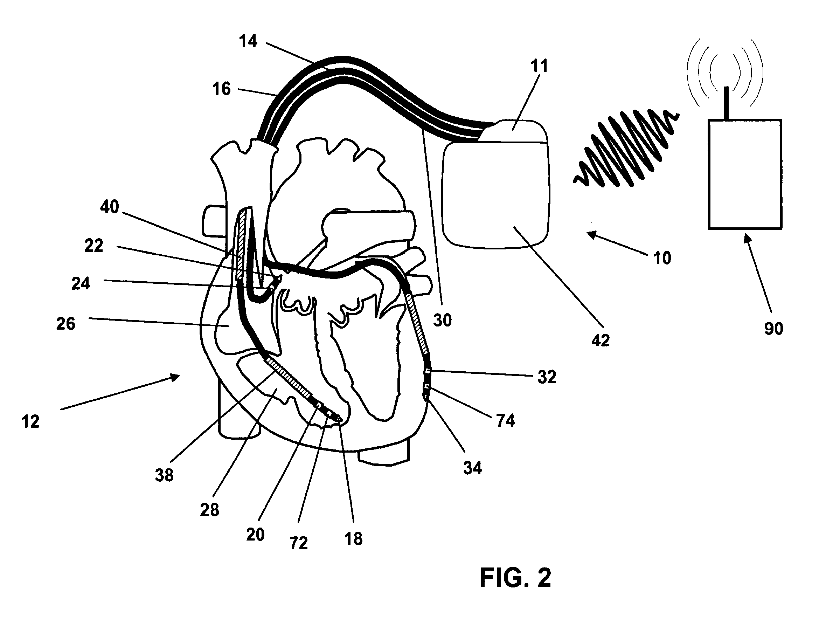

Figur 2 zeigt den implantierbaren Herzstimulator 10 in Form eines Dreikammerherzschrittmachers/ Kardiovertes/Defibrillators mit daran angeschlossenen Elektrodenleitungen 14, 16 und 30 in Verbindung mit einem Herz 12. Außerdem ist noch einmal das externe Gerät 90 in der Nähe des implantierbaren Herzstimulators 10 dargestellt. Die Elektrodenleitungen 14, 16 und 30 sind über bekannte standardisierte Steckverbindungen mit Kontaktbuchsen in einem Header (Anschlussgehäuse) 11 des Herzstimulators 10 elektrisch verbunden. Auf diese Weise sind die Elektrodenleitungen 14, 16 und 30 auch mit elektronischen Komponenten im Inneren eines hermetisch dichten Metallgehäuses 42 des Herzstimulators 10 verbunden. Diese Komponenten sind nachfolgend detaillierter schematisch dargestellt und bestimmen die erfindungsgemäße Funktionsweise des Herzstimulators 10.

Die Elektrodenleitung 14 ist eine rechtsatriale Elektrodenleitung und besitzt an ihrem distalen Ende eine atriale Spitzenelektrode RA Tip 22 sowie in kurzer Entfernung davon eine atriale Ringelektrode RA Ring 24, die beide im rechten Atrium 26 des Herzens 12 platziert sind.

Die Elektrodenleitung 16 ist eine rechtsventrikuläre Elektrodenleitung und besitzt an ihrem distalen Ende eine rechtsventrikuläre Spitzenelektrode RV Tip 18 und in unmittelbarer Nähe eine rechtsventrikuläre Ringelektrode RV Ring 20. Beide Elektroden sind im Apex des rechten Ventrikels 28 des Herzens 12 angeordnet.

Außerdem ist die rechtsventrikuläre Elektrodenleitung 16 noch eine rechtsventrikuläre Schockwendel RV Schock 38 als großflächige Elektrode zur Abgabe von Defibrillationsschocks. Eine weitere Schockwendel 40 ist in der Vena cava superior angeordnet und wird deshalb nachfolgend auch als SVC-Schockelektrode bezeichnet.

Die Elektrodenleitung 30 ist eine linksventrikuläre Elektrodenleitung, an deren distalem Ende eine linksventrikuläre Spitzenelektrode LV Tip 34 sowie in deren Nähe eine linksventrikuläre Ringelektrode LV Ring 32 angeordnet ist. Außerdem trägt die linksventrikuläre Elektrodenleitung 30 eine nicht näher bezeichnete, aber in Figur 2 dargestellte linksventrikuläre Schockwendel zur Abgabe von Defibrillationsschocks an den linken Ventrikel. Die linksventrikuläre Elektrodenleitung 30 wird vom rechten Atrium 26 des Herzens 12 aus über den Koronarsinus in eine von diesem abzweigende Lateralvene geführt und wird deswegen auch als Koronarsinuselektrodenleitung oder CS-Elektrodenleitung bezeichnet.

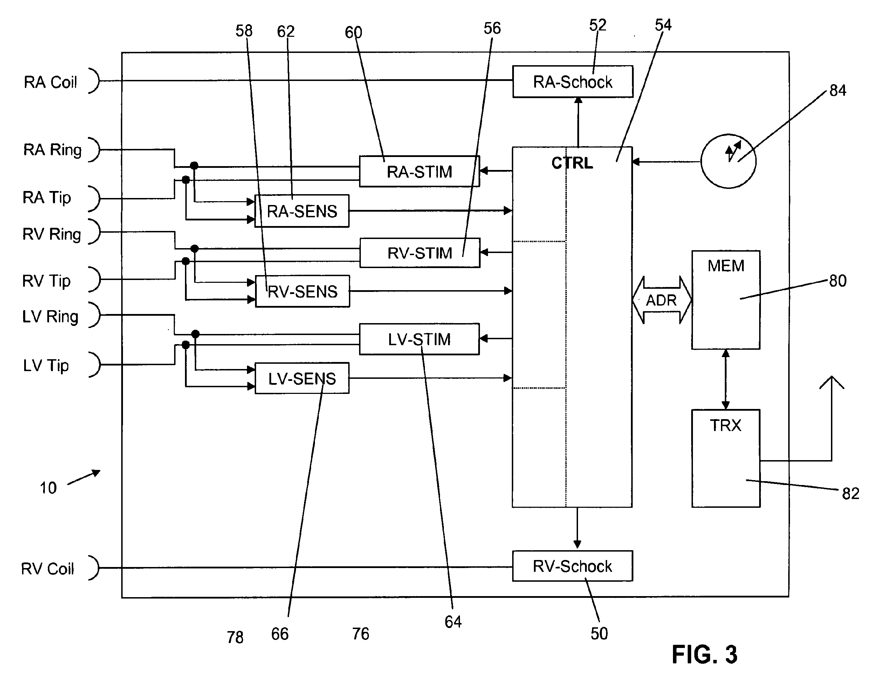

In Figur 3 sind die Hauptbestandteile des Herzstimulators 10 dargestellt. Auf der linken Seite sind die elektrischen Anschlüsse für die verschiedenen Elektroden 18, 20, 22, 24, 32, 34, 38 und 40 dargestellt. Die Schockelektroden 38 und 40 sind jeweils mit einem rechtsventrikulären Schockimpulsgenerator 50 bzw. SVC-Schockgenerator 52 verbunden. Beide Schockgeneratoren 50 und 52 sind mit einer Stimulationssteuereinheit 54 verbunden, die die beiden Schockimpulsgeneratoren 50 und 52 bei Bedarf zur Erzeugung und Abgabe eines Defibrillationsschocks ansteuert.

Der Anschluss für die rechtsventrikuläre Tippelektrode RV Tip sowie der Anschluss für die rechtsventrikuläre Ringelektrode RV Ring sind jeweils sowohl mit einer rechtsventrikulären Stimulationseinheit 56 als auch mit einer rechtsventrikulären Sensing-Einheit 58 verbunden. Sowohl die rechtsventrikuläre Stimulationseinheit 56 als auch die rechtsventrikuläre Sensing-Einheit 58 sind jeweils mit der Stimulationssteuereinheit 54 verbunden.

Die rechtsventrikuläre Stimulationseinheit 56 ist dazu ausgebildet, auf ein Ansteuersignal der Stimulationssteuereinheit 54 hin einen rechtsventrikulären Stimulationsimpuls zu erzeugen und im Anschluss an die rechtsventrikuläre Ringelektrode und die rechtsventrikuläre Tippelektrode abzugeben. Alternativ ist es auch möglich, dass das Gehäuse 42 des Herzstimulators 10 eine neutrale Elektrode bildet und die rechtsventrikuläre Stimulationseinheit 56 mit dem Anschluss für die rechtsventrikuläre Ringelektrode RV Tip und dem Gehäuse 42 als andere Elektrode zur Abgabe eines Stimulationsimpulses verbunden ist. Ein rechtsventrikulärer Stimulationsimpuls unterscheidet sich von einem Defibrillationsschock dadurch, dass der Stimulationsimpuls eine wesentlich geringere Impulsstärke besitzt, so dass er nicht wie ein Defibrillationsschock auf einen Schlag das vollständige Herzgewebe (Myokard) einer Herzkammer erregt, sondern nur die Herzmuskelzellen in unmittelbarer Umgebung der rechtsventrikulären Spitzenelektrode 18. Diese Erregung breitet sich dann durch natürliche Reizleitung über den gesamten Ventrikel weiter aus und sorgt so für eine stimulierte Kontraktion des Ventrikels.

Die rechtsventrikuläre Sensing-Einheit 58 ist dazu ausgebildet, an dem Anschluss für die rechtsventrikuläre Ringelektrode RV Ring und die rechtsventrikuläre Tippelektrode RV Tip anliegende elektrische Potentiale zunächst durch einen Eingangsverstärker zu verstärken und zu filtern. Weiterhin ist die rechtsventrikuläre Sensing-Einheit ausgebildet, den Verlauf der an ihren Eingängen anliegenden elektrischen Signale derart auszuwerten, dass die rechtsventrikuläre Sensing-Einheit 58 selbsttätig eine natürliche (intrinsische), d.h. selbsttätige Kontraktion des rechten Ventrikels detektiert. Dies kann beispielsweise dadurch geschehen, dass der Verlauf des an den Eingängen der rechtsventrikulären Sensing-Einheit 58 anliegenden Signals mit einem Schwellwert verglichen wird. Typischerweise ist die größte Amplitude des Signals in Form der sogenannten R Zacke kennzeichnend für eine natürliche Kontraktion des rechten Ventrikels, die durch Schwellwertvergleich detektiert werden kann. Die rechtsventrikuläre Sensing-Einheit 58 gibt daraufhin ein entsprechendes, eine natürliche Kontraktion des rechten Ventrikels anzeigendes Ausgangssignal an die Stimulationssteuereinheit 54 aus.

In analoger Weise sind der Anschluss für die rechtsatriale Tippelektrode und der Anschluss für die rechtsatriale Ringelektrode sowohl mit einer rechtsatrialen Stimulationseinheit 60 als auch mit einer rechtsatrialen Sensing-Einheit 62 verbunden, die jeweils ihrerseits wiederum mit der Stimulationssteuereinheit 54 verbunden sind. Die rechtsatriale Stimulationseinheit 60 ist dazu ausgebildet, Stimulationsimpulse zu erzeugen, deren Stärke ausreicht, das rechtsatriale Myokard zu erregen. Die rechtsatrialen Stimulationsimpulse können dabei eine andere Impulsstärke besitzen als die rechtsventrikulären Stimulationsimpulse. Die rechtsatriale Sensing-Einheit 62 ist ausgebildet, aus dem Verlauf des an ihren Eingängen anliegenden Differenzsignals eine sogenannte P-Welle zu detektieren, die eine natürliche (intrinsische) Kontraktion des rechten Atriums kennzeichnet. Detektiert die rechtsatriale Sensing-Einheit 62 eine entsprechende P-Welle, erzeugt sie ein Ausgangssignal und gibt dieses an die Stimulationssteuereinheit 54 weiter, welches eine natürliche Kontraktion des rechten Atriums kennzeichnet.

In gleicher Weise sind auch der Anschluss für die linksventrikuläre Tippelektrode LV Tip und der Anschluss für die linksventrikuläre Ringelektrode LV Ring mit einer linksventrikulären Stimulationseinheit 64 und einer linksventrikulären Sensing-Einheit 66 verbunden. Die linksventrikuläre Stimulationseinheit 64 und die linksventrikuläre Sensing-Einheit 66 sind ebenso mit der Stimulationssteuereinheit 54 verbunden. Beide funktionieren ähnlich wie die bereits beschriebenen Stimulationseinheiten 56 und 60 und Sensing-Einheiten 58 und 62.

Weiterhin umfasst der Herzstimulator 10 eine Speichereinheit 80, die mit der Stimulationssteuereinheit 54 verbunden ist und es erlaubt, von der Stimulationssteuereinheit 54 erzeugte oder ausgewertete Signale zu speichern. Andererseits erlaubt es die Speichereinheit 80, Steuerprogramme für die Stimulationssteuereinheit 54 in veränderbarer Form zu speichern. Die Speichereinheit 80 dient im Rahmen der hier vorgestellten Erfindung insbesondere dazu, Werte jüngster intrinsischer RR-Intervalle sowie wenigstens einen Wert für eine interventrikuläre Verzögerungszeit VVD zu speichern.

Des Weiteren ist die Stimulationssteuereinheit 54 mit einem Zeitgeber 84 verbunden, der es der Stimulationssteuereinheit 54 unter anderem ermöglicht Zeitpunkte und Zeitintervalle zu bestimmen.

Die Speichereinheit 80 ist mit einer Telemetrie-Einheit 82 verbunden, die es erlaubt, in der Speichereinheit 80 abgelegte Daten drahtlos an das externe Gerät 100 zu übertragen oder Programmierbefehle seitens des externen Geräts 100 zu dem Herzstimulator 10 zu übertragen und in der Speichereinheit 80 zu speichern.

Als Dreikammerherzstimulator/Kardioverter/Defibrillator ist der Herzstimulator 10 in der Lage, eine Stimulation des rechten Atriums, des rechten Ventrikels und des linken Ventrikels oder auch nur einer oder zweier dieser Herzkammern in an sich bekannter Weise durchzuführen. Dies schließt insbesondere die Stimulation einer jeweiligen Herzkammer im Demand-Modus ein, in der Stimulationsimpulse nur dann an die jeweilige Herzkammer abgegeben werden, falls in einem vorangehenden jeweiligen Escape-Intervall seitens der jeweiligen Sensing-Einheit keine intrinsische Kontraktion der jeweiligen Herzkammer erfasst wird. Damit ist der Herzschrittmacher in der Lage, die bekannten rechtsventrikulären Stimulationsmodi wie VVI, VVD oder DDD durchzuführen.

Für das Timing der Stimulationsimpulse im biventrikulären Stimulationsmodus, in dem beide Ventrikel eines Herzens stimuliert werden, ist insbesondere eine interventrikuläre Verzögerungszeit (VV-Intervall; VVD) von Bedeutung, d.h. die Zeit, mit der ein rechter Stimulationsimpuls und ein linker Stimulationsimpuls (sofern sie im Demand-Modus nicht inhibiert werden) aufeinanderfolgen. Diese Zeit kann kleiner als 0 sein, so dass der linke Stimulationsimpuls dem rechten Stimulationsimpuls nachfolgt. Die interventrikuläre Verzögerungszeit kann 0 sein, was bedeutet, dass ein rechtsventrikulärer Stimulationsimpuls und ein linksventrikulärer Stimulationsimpuls durch gleichzeitige Ansteuerung der rechtsventrikulären Stimulationseinheit 56 und der linksventrikulären Stimulationseinheit 64 durch die Stimulationssteuereinheit 54 gleichzeitig abgegeben werden. Die interventrikuläre Verzögerungszeit kann auch größer als 0 sein, was bedeutet, dass ein linksventrikulärer Stimulationsimpuls vor der Abgabe des zugehörigen rechtsventrikulären Stimulationsimpulses abgegeben wird.

Die Stimulationssteuereinheit 54 ist ausgebildet, eine Resynchronisation von rechtem und linkem Ventrikel unter Verwendung eines Implantates und mindestens einer Elektrode im rechten sowie im linken Ventrikel zu bewirken. Stimulationen in einem der beiden Ventrikel werden durch die Stimulationssteuereinheit 54 unterdrückt, wenn die jeweilige Sensing-Einheit dort ein intrinsisches Ereignis erfasst.

Zur Resynchronisation bestimmt die Stimulationssteuereinheit 54 ein Verzögerungsintervall Δ (siehe Figur 4), das Schlag für Schlag an die Dauer des letzten intrinsischen RR-Intervalls angepasst wird. Eine Optimierung findet dabei mit Hilfe einer z.B. hämodynamischen Ersatzgröße durch die Anpassung eines Parameters VVD [ms] statt. Dieser Parameter VVD entspricht einer interventrikulären Verzögerungszeit und gibt an, um wieviel der zu resynchronisierende Ventrikel vor der nächsten erwarteten intrinsischen Erregung stimuliert werden soll.

Der Stimulationszeitpunkt in einem der beiden Ventrikel folgt somit der jeweiligen Variabilität der Herzfrequenz und des intrinsischen AV-Delays.

Ein geeigneter Wert für VVD ist in dem Speicher 80 gespeichert. Er kann beispielsweise von einem Arzt während eines Follow-up bestimmt werden. Dabei wird VVD variiert, um ein hämodynamisches Optimum, abgeleitet aus z.B. intrakardialer Impedanz, Drucksensoren oder bildgebenden Verfahren zu suchen.

Für den Fall, dass eine intrinsische Erregung des rechten Ventrikels nicht detektiert wird, weil sie in die Blanking-Zeit nach der ventrikulären Stimulation fällt und dadurch eine direkte Bestimmung des Verzögerungsintervalls Δ nicht möglich ist, bestimmt die Stimulationssteuereinheit 54 das Verzögerungsintervall Δ als Differenz zwischen der letzten intrinsischen Erregung und der Mitte des Blanking-Intervalles.

Wenn das nicht zum Erfolg führt, unterdrückt die Stimulationssteuereinheit 54 eine Stimulation des linken Ventrikels für einen Herzschlag, um die intrinsische RR zu vermessen und daraus ein neues Verzögerungsintervall Δ zu bestimmen.

Die Stimulationssteuereinheit 54 ist außerdem ausgebildet, in regelmäßigen Abständen eine direkte Überleitung vom linken in den rechten Ventrikel (oder umgekehrt) dadurch zu erkennen, dass sie das Verzögerungsintervall Δ für einen oder mehrere Herzzyklen um τ verringert und die resultierende Veränderung des RR-Intervalls misst. Wenn sich RR ebenfalls um τ verändert, wird eine direkte Überleitung detektiert. In diesem Fall wird der gespeicherte Wert für die interventrikuläre Verzögerungszeit VVD reduziert.

Figur 4 zeigt ein Beispiel für die Stimulation des linken Ventrikels mittels eines Herzstimulators gemäß der Erfindung bei zeitweise intakter natürlicher Kontraktion des rechten Ventrikels. Im oberen Teil sind die linksventrikulären, im unteren Teil die rechtsventrikulären Ereignisse dargestellt, wobei mit Vp stimulierte Ereignisse und mit Vs intrinsische Ereignisse bezeichnet sind.

Die Stimulationssteuereinheit 54 verwendet die Periodendauer von zwei aufeinander folgenden intrinsischen rechts- oder linksventrikulären Kontraktionen - erfasst durch die jeweilige Sensing-Einheit 58 oder 66 - zur Bestimmung eines optimalen Verzögerungsintervalls Δ zwischen einem rechtsventrikulären Ereignis und einem linksventrikulären Pace Ereignis, also dem Zeitpunkt, zu dem ein linksventrikulärer Stimulationsimpuls abgegeben werden soll.

Das erste bestimmte intrinsische RR-Intervall RR1 wird zusammen mit einem gespeicherten Wert für die interventrikuläre Verzögerungszeit VVD zum Bestimmen des ersten Verzögerungsintervalls $\Delta_1$ genutzt:

$$\Delta_1 = RR1 - VVD \ [ms]$$

Analog wird aus dem nächsten intrinsische RR-Intervall RR1 das nächste Verzögerungsintervall $\Delta_2$ bestimmt und damit der Zeitpunkt des nächstens linksventrikulären Stimulationsimpulses:

$$\Delta_2 = RR2 - VVD \ [ms];$$

Im gewählten Beispiel erfolgt die Stimulation des linken Ventrikels zeitgleich mit der rechten intrinsischen Erregung, weil sich die Herzfrequenz (HF) erhöht hat. Entsprechend wird die nächste Stimulation im linken Ventrikel, der höheren HF folgend, früher stimuliert

$$\Delta_3 = RR3 - VVD \ [ms]$$

Im Beispiel ist das nächste intrinsische R-Intervall wieder länger, weil sich die Herzfrequenz verlangsamt hat. Dadurch verlängert sich auch automatisch das Verzögerungsintervall $\Delta_4$ zwischen dem nächstem intrinsischen Ereignis und der Stimulation des linken Ventrikels:

$$\Delta_4 = RR4 - VVD \ [ms]$$

Mit der Bildung des nächsten Verzögerungsintervalls $\Delta_5$ ist die Anpassung an die verlangsamte Herzfrequenz abgeschlossen:

$$\Delta_5 = RR5 - VVD \ [ms]$$

**Patentansprüche**

1. Implantierbarer Herzstimulator (10) mit wenigstens einer ersten Sensing-Einheit (58) zum Erfassen von Eigenaktionen eines ersten Ventrikels, die einen Eingang aufweist, der mit einer ersten Elektrodenleitung (16) zur Aufnahme von Signalen eines ersten Ventrikels verbunden oder zu verbinden ist und die ausgebildet ist, ein an ihrem Eingang anliegendes elektrisches Eingangssignal derart auszuwerten, dass die Sensing-Einheit wenigstens ein für eine Kontraktion des ersten Ventrikels typisches Signalmerkmal detektiert und ein entsprechendes Ausgangssignal erzeugt, und wenigstens einer ventrikulären Stimulationseinheit (64) zum Stimulieren eines jeweils anderen, zweiten Ventrikels eines Herzens, die einen Ausgang aufweist, der mit einer zweiten Elektrodenleitung (30) zur Stimulation des anderen, zweiten Ventrikels verbunden oder zu verbinden ist und die ausgebildet ist, auf ein Steuersignal hin einen ventrikulären Stimulationsimpuls zu erzeugen und über den Ausgang abzugeben, sowie einer Stimulationssteuereinheit (54), die mit der ersten Sensing-Einheit (58) und der Stimulationseinheit (64) verbunden und ausgebildet ist, Ausgangssignale der ersten Sensing-Einheit (58) zu verarbeiten und Steuersignale für die Stimulationseinheiten (64) zu erzeugen, Vwobei die Stimulationssteuereinheit (54) ausgebildet ist, aus erfassten ventrikulären Eigenaktionen R des ersten Ventrikels ein jeweils aktuelles intrinsisches RR-Intervall abzuleiten und aus dem so gebildeten RR-Intervall ein Verzögerungsintervall $\Delta$ unter Berücksichtigung eines Wertes für eine interventrikuläre Verzögerungszeit VVD als $\Delta = RR - VVD$ zu bestimmen, wobei das Verzögerungsintervall $\Delta$ mit einem ventrikulären Ereignis des ersten Ventrikels beginnt und zu dessen Ende die Stimulationssteuereinheit (54) eine Stimulation des zweiten Ventrikels auslöst,
**dadurch gekennzeichnet, dass** die Stimulationssteuereinheit ausgebildet ist, eine direkte Überleitung vom linken in den rechten Ventrikel oder umgekehrt dadurch zu erkennen, dass sie

   - das Verzögerungsintervall $\Delta$ für einen oder mehrere Herzzyklen um eine Zeitdauer $\tau$ verringert,
   - eine resultierende Veränderung des RR-Intervalls bestimmt
   - und dann, wenn sich das RR-Intervall ebenfalls um $\tau$ verändert, eine direkte Überleitung detektiert und einen gespeicherten Wert für die interventrikuläre Verzögerungszeit VVD reduziert.

2. Herzstimulator nach Anspruch 1, **dadurch gekennzeichnet, dass** die erste Sensing-Einheit eine rechtsventrikuläre Sensing-Einheit (58) ist, die einen Eingang aufweist, der mit einer rechtsventrikulären Elektrodenleitung (16) verbunden oder zu verbinden ist und die ausgebildet ist, ein an ihrem Eingang anliegendes elektrisches Eingangssignal derart auszuwerten, dass die rechtsventrikuläre Sensing-Einheit wenigstens ein für eine Kontraktion eines rechten Ventrikels typisches Signalmerkmal detektiert und ein entsprechendes Ausgangssignal erzeugt,
und dass die erste Stimulationseinheit eine linksventrikuläre Stimulationseinheit (64) ist, die einen Ausgang aufweist, der mit einer linksventrikulären Elektrodenleitung (30) verbunden oder zu verbinden ist und die ausgebildet ist, auf ein Steuersignal hin einen linksventrikulären Stimulationsimpuls zu erzeugen und über den Ausgang abzugeben.

3. Herzstimulator nach Anspruch 1, **dadurch gekennzeichnet, dass** die erste Sensing-Einheit eine linksventrikuläre Sensing-Einheit (66) ist, die einen Eingang aufweist, der mit einer linksventrikulären Elektrodenleitung (30) verbunden oder zu verbinden ist und die ausgebildet ist, ein an ihrem Eingang anliegendes elektrisches Eingangssignal derart auszuwerten, dass die linksventrikuläre Sensing-Einheit wenigstes ein für eine Kontraktion eines linken Ventrikels typisches Signalmerkmal detektiert und ein entsprechendes Ausgangssignal erzeugt, und dass die erste Stimulationseinheit eine rechtsventrikuläre Stimulationseinheit (56) ist, die einen Ausgang aufweist, der mit einer rechtsventrikulären Elektrodenleitung (16) verbunden oder zu verbinden ist und die ausgebildet ist, auf ein Steuersignal hin einen rechtsventrikulären Stimulationsimpuls zu erzeugen und über den Ausgang abzugeben.

4. Herzstimulator nach einem der Ansprüche 1 bis 3, **dadurch gekennzeichnet, dass** der Herzstimulator jeweils eine rechtsventrikuläre und eine linksventrikuläre Sensing-Einheit sowie jeweils eine rechtsventrikuläre und eine linksventrikuläre Stimulationseinheit aufweist.

5. Herzstimulator nach einem der Ansprüche 1 bis 4, **dadurch gekennzeichnet, dass** das RR-Intervall aus mehreren vorhergehenden RR-Intervallen bestimmt wird.

6. Herzstimulator nach einem der Ansprüche 1 bis 5, **dadurch gekennzeichnet, dass** die Stimulationssteuereinheit ausgebildet ist, für den Fall, dass eine intrinsische Erregung nicht detektiert wird, weil sie in die Blanking-Zeit nach der ventrikulären Stimulation fällt und dadurch eine direkte Bestimmung des Verzögerungsintervalls $\Delta$ nicht möglich ist,

    - das Verzögerungsintervall $\Delta$ als Differenz zwischen der letzten intrinsischen Erregung und der Mitte des Blanking-Intervalls zu bestimmen,
    - eine Stimulation des zweiten Ventrikels für einen Herzschlag zu unterdrücken, das intrinsische RR-Intervall zu bestimmen und daraus ein neues Verzögerungsintervall $\Delta$ zu bestimmen, und
    - das Verzögerungsintervall $\Delta$ so lange zu reduzieren, bis wieder eine intrinsische Erregung detektiert werden kann.

7. Herzstimulator nach einem der Ansprüche 1 bis 6, **dadurch gekennzeichnet, dass** der Herzstimulator dazu ausgebildet ist, selbsttätig eine atrioventrikuläre Verzögerungszeit zwischen einem rechtsatrialen Ereignis und einem rechtsventrikulären Ereignis zu bestimmen und die Stimulationssteuereinheit dazu ausgebildet ist, das Verzögerungsintemall VVD unter Berücksichtigung der von dem Herzstimulator bestimmten atrioventrikulären Verzögerungszeit zu bilden.

## Claims

1. An implantable cardiac stimulator (10) comprising at least one first sensing unit (58) for detecting natural actions of a first ventricle, which has a first input which is connected or connectable to a first electrode lead (16) for receiving signals from a first venticle, and which is designed to evaluate an electrical input signal present at the input thereof in such a way that the sensing unit detects at least one signal feature which is typical for a contraction of the first ventricle and generates a corresponding output signal, and at least one ventricular stimulation unit (64) for stimulating another, second ventricle of a heart, which comprises an input which is connected or connectable to a second electrode lead (30) for stimulating the other, second ventricle, and which is designed to generate a ventricular stimulation pulse in response to a control signal and deliver it via an output, and a stimulation control unit (54) which is connected to the first sensing unit (58) and the stimulation unit (64), and is designed to process output signals of the first sensing unit (58) and generate control signals for the stimulation units (64), wherein the stimulation control unit (54) is designed to derive a current intrinsic RR interval from detected ventricular natural actions R of the first ventricle and, on the basis of the thusly formed RR interval, to determine a delay interval $\Delta$ with consideration for a value for an interventricular delay time VVD as $\Delta = RR - VVD$, wherein the delay interval $\Delta$ starts with a ventricular event of the first ventricle and, at the end thereof, the stimulation control unit (54) initiates a stimulation of the second ventricle, **characterized in that** the stimulation control unit is designed to detect a direct transmission from the left ventricle into the right ventricle, or vice versa, **in that** it

    - reduces the delay interval $\Delta$ for one or more cardiac cycles by a period $\tau$,
    - determines a resulting change in the RR interval
    - and then, when the RR interval likewise changes by $\tau$, detects a direct transmission and reduces a stored value for the interventricular delay time VVD.

2. The cardiac stimulator according to claim 1, **char-**

acterized in that the first sensing unit is a right-ventricular sensing unit (58) which comprises an input which is connected or connectable to a right-ventricular electrode lead (16), and is designed to evaluate an electrical input signal present at the input thereof in such a way that the right-ventricular sensing unit detects at least one signal feature which is typical for a contraction of a right ventricle and generates a corresponding output signal, and the first stimulation unit is a left-ventricular stimulation unit (64) which has an input which is connected or connectable to a left-ventricular electrode lead (30) and is designed to generate a left-ventricular stimulation pulse in response to a control signal and deliver it via an output.

3. The cardiac stimulator according to claim 1, characterized in that the first sensing unit is a left-ventricular sensing unit (66) which comprises an input which is connected or connectable to a left-ventricular electrode lead (30), and is designed to evaluate an electrical input signal present at the input thereof in such a way that the left-ventricular sensing unit detects at least one signal feature which is typical for a contraction of a left ventricle and generates a corresponding output signal, and the first stimulation unit is a right-ventricular stimulation unit (56) which has an input which is connected or connectable to a right-ventricular electrode lead (16) and is designed to generate a right-ventricular stimulation pulse in response to a control signal and deliver it via an output.

4. The cardiac stimulator according to one of the claims 1 to 3, characterized in that the cardiac stimulator comprises a right-ventricular sensing unit and a left-ventricular sensing unit, and a right-ventricular stimulation unit and a left-ventricular stimulation unit.

5. The cardiac stimulator according to one of the claims 1 to 4, characterized in that the RR interval is determined on the basis of a plurality of preceding RR intervals.

6. The cardiac stimulator according to one of the claims 1 to 5, characterized in that the stimulation control unit is designed to do the following in the case that an intrinsic excitation is not detected because it falls in the blanking time after the ventricular stimulation and, therefore, direct determination of the delay interval Δ is not possible,

- determine the delay interval Δ as the difference between the last intrinsic excitation and the middle of the blanking interval,
- suppress a stimulation of the second ventricle for a heartbeat, determine the intrinsic RR interval and, on the basis thereof, determine a new delay interval Δ, and
- reduce the delay interval Δ until an intrinsic excitation can be detected once more.

7. The cardiac stimulator according to one of the claims 1 to 6, characterized in that the cardiac stimulator is designed to automatically determine an atrioventricular delay time between a right-atrial event and a right-ventricular event, and the stimulation control unit is designed to calculate the delay interval VVD with consideration for the atrioventricular delay time determined by the cardiac stimulator.

**Revendications**

1. Stimulateur cardiaque implantable (10) avec

- au moins une première unité de détection (58) pour la détection d'actions propres d'un premier ventricule, comportant une entrée reliée ou à relier avec une première ligne d'électrodes (16) pour la réception de signaux d'un premier ventricule, et conçue pour évaluer un signal d'entrée électrique appliqué à son entrée, de telle manière que l'unité de détection détecte au moins une caractéristique de signal typique pour une contraction du premier ventricule et produit un signal de sortie correspondant, et au moins une unité de stimulation ventriculaire (64) pour stimuler un autre deuxième ventricule d'un coeur, comportant une sortie reliée ou à relier à une deuxième ligne d'électrodes (30) pour stimuler l'autre deuxième ventricule, et conçue pour produire une impulsion de stimulation ventriculaire en réponse à un signal de commande et délivrer celle-ci par la sortie, ainsi qu'avec une unité de commande de stimulation (54) reliée à la première unité de détection (58) et à l'unité de stimulation (64) et conçue pour traiter les signaux de sortie de la première unité de détection (58) et produire des signaux de commande pour les unités de stimulation, l'unité de stimulation (54) étant conçue pour dériver un intervalle RR intrinsèque respectivement actuel à partir des actions ventriculaires propres R du premier ventricule, et pour déterminer un intervalle de retard Δ sous la forme Δ = RR - VVD, à partir de l'intervalle RR ainsi formé, en tenant compte d'une valeur VVD pour un temps de retard inter-ventriculaire, l'intervalle de retard Δ commençant avec un évènement ventriculaire du premier ventricule, et l'unité de commande de stimulation (54) déclenchant une stimulation du deuxième ventricule à la fin de celui-ci,
caractérisé en ce que l'unité de commande de stimulation est conçue pour reconnaître une déviation directe du ventricule gauche au ventricule droit et inversement, par le fait qu'elle
- réduit l'intervalle de retard Δ d'une durée τ pour un ou plusieurs cycles cardiaques,
- détermine une modification résultante de l'intervalle RR,
- puis, lorsque l'intervalle RR se modifie également de τ, détecte une déviation directe et réduit une valeur enregistrée pour le temps de retard inter-ventriculaire VVD.

2. Stimulateur cardiaque selon la revendication 1, **caractérisé en ce que** la première unité de détection est une unité de détection de ventricule droit (58) comportant une entrée reliée ou à relier à une ligne d'électrodes de ventricule droit (16), et conçue pour évaluer un signal d'entrée électrique appliqué à son entrée, de telle manière que l'unité de détection de ventricule droit détecte au moins une caractéristique de signal typique pour une contraction d'un ventricule droit et produit un signal de sortie correspondant,
et **en ce que** la première unité de stimulation est une unité de stimulation de ventricule gauche (64) comportant une sortie reliée ou à relier à une ligne d'électrodes de ventricule gauche (30), et conçue pour produire une impulsion de stimulation de ventricule gauche en réponse à un signal de commande et délivrer celle-ci par la sortie.

3. Stimulateur cardiaque selon la revendication 1, **caractérisé en ce que** la première unité de détection est une unité de détection de ventricule gauche (66), comportant une entrée reliée ou à relier à une ligne d'électrodes de ventricule gauche (30), et conçue pour évaluer un signal d'entrée électrique appliqué à son entrée, de telle manière que l'unité de détection de ventricule gauche détecte au moins une caractéristique de signal typique pour une contraction d'un ventricule gauche et produit un signal de sortie correspondant, et **en ce que** la première unité de stimulation est une unité de stimulation de ventricule droit (56), comportant une sortie 16) reliée ou à relier à une ligne d'électrodes de ventricule droit (16), et conçue pour produire une impulsion de stimulation de ventricule droit en réponse à un signal de commande et délivrer celle-ci par la sortie.

4. Stimulateur cardiaque selon l'une des revendications 1 à 3, **caractérisé en ce que** le stimulateur cardiaque comporte respectivement une unité de détection de ventricule droit et une unité de détection de ventricule gauche, ainsi qu'une unité de stimulation de ventricule droit et une unité de stimulation de ventricule gauche.

5. Stimulateur cardiaque selon l'une des revendications 1 à 4, **caractérisé en ce que** l'intervalle RR est déterminé à partir de plusieurs intervalles RR précédents.

6. Stimulateur cardiaque selon l'une des revendications 1 à 5, **caractérisé en ce que** l'unité de commande de stimulation est conçue, dans le cas où une excitation intrinsèque ne serait pas détectée, du fait qu'elle tombe pendant le temps d'extinction après la stimulation ventriculaire et qu'une détermination directe de l'intervalle de retard Δ n'est donc pas possible,

- pour déterminer l'intervalle de retard Δ comme la différence entre la dernière excitation intrinsèque et le milieu de l'intervalle d'extinction,
- pour empêcher une stimulation du deuxième ventricule pour un battement de coeur, déterminer l'intervalle RR intrinsèque et déterminer un nouvel intervalle de retard Δ à partir de celui-ci,
- réduire l'intervalle de retard Δ jusqu'à ce qu'une excitation intrinsèque puisse à nouveau être détectée.

7. Stimulateur cardiaque selon l'une des revendications 1 à 6, **caractérisé en ce que** le stimulateur cardiaque est conçu pour déterminer automatiquement un temps de retard atrio-ventriculaire entre un évènement atrial droit et un évènement ventriculaire droit, et **en ce que** l'unité de stimulation est conçue pour former l'intervalle de retard VVD en tenant compte du temps de retard atrio-ventriculaire déterminé par le stimulateur cardiaque.

FIG. 1

FIG. 2

FIG. 3

MEM — 80

TRX — 82

ADR

CTRL — 54

RA-Schock — 52

RV-Schock — 50

RA-STIM — 56

RV-STIM

LV-STIM — 64

RA-SENS — 62

RV-SENS — 58

LV-SENS — 66

60

76

78

84

RA Coil

RA Ring

RA Tip

RV Ring

RV Tip

LV Ring

LV Tip

10

RV Coil

**FIG. 4**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2060299 A
- US 2008009909 A1
- US 2004215257 A1